# EUROPEAN PATENT APPLICATION

(11) **EP 4 653 033 A1**
(43) Date of publication of application: **26.11.2025**
(21) Application number: 23921945.4
(22) Date of filing: 17.02.2023
(51) Int. Cl.: A61M 5/32

(54) **SAFETY INSULIN PEN NEEDLE**

(71) Applicant: CMT Health Pte. Ltd., Singapore 189720 (SG)
(72) Inventor: LU, Jun, Wuxi, Jiangsu 214400 (CN); LU, Weiwei, Wuxi, Jiangsu 214400 (CN); ZHAO, Lijie, Wuxi, Jiangsu 214400 (CN); NI, Lin, Wuxi, Jiangsu 214400 (CN); CHEN, Tianzhu, Wuxi, Jiangsu 214400 (CN)
(74) Representative: Buzzi, Notaro & Antonielli d'Oulx S.p.A.
(86) International application number: PCT/CN2023/076707
(87) International publication number: WO 2024/168793

(57) **Abstract**

Provided is a safety insulin pen needle. The safety insulin pen needle includes a needle hub, a protective sleeve, a needle cap, and a limiting block. A fixed post protruding along with an injection needle is disposed on the needle hub. A snap-fit step is disposed on the surface of the fixed post. The injection needle is inserted through the protective sleeve. A spring is clamped between the protective sleeve and the needle hub such that the protective sleeve is axially movable relative to the needle hub to expose or cover a needle tip of the injection needle. A protruding edge is disposed on an outer wall of the protective sleeve. A first limiting step and a second limiting step are disposed on an inner wall of the protective sleeve along a direction in which the injection needle penetrates. The inner diameter of the second limiting step is smaller than the inner diameter of the first limiting step. The needle cap is fixedly assembled with the needle hub and encloses the protective sleeve within the needle cap. The needle cap is provided with a through-hole allowing the protective sleeve to extend out. The through-hole limits the protruding edge. The limiting block is located in an inner cavity of the protective sleeve and is sleeved on the injection needle. A first end of the limiting block is provided with a claw that engages with the snap-fit step, and a second end of the limiting block cooperates with the first limiting step or the second limiting step to stop the protective sleeve.

## Description

### TECHNICAL FIELD

This application relates to the technical field of medical devices, for example, to a safety insulin pen needle.

### BACKGROUND

Insulin injection devices are widely used for treating diabetes. The primary function of an insulin injection device is to accurately deliver insulin into the body of a diabetic patient to control blood glucose levels.

The traditional injection form is an injection needle structure. The injection device includes a needle hub, a needle tube, and an outer protective housing. Before use, the protective housing is removed, leaving the insulin injection needle exposed. After use, due to the absence of a safety protection structure for the injection needle, accidental contact with the needle tip may easily occur, leading to cross-infection. Additionally, without necessary protective structures, such injection needles may be reused after initial use, posing a risk of repeated use.

There exists a type of insulin pen needle that offers portable, pen-like usage and includes a locking or destruction mechanism after use to ensure single-use functionality. However, the structures of such products are complex and varied, with diverse operations such as twisting, screwing, squeezing, or pressing. Users need to specifically study the instructions before use, resulting in a poor user experience. Moreover, the complex structures increase manufacturing costs, adding to the economic burden on patients.

### SUMMARY

This application provides a safety insulin pen needle to achieve single-use functionality of an injection needle, reduce operational complexity, and improve safety and convenience of use.

This application adopts technical solutions described below.

A safety insulin pen needle includes a needle hub, a protective sleeve, a needle cap, and a limiting block.

A fixed post protruding along with an injection needle is disposed on the needle hub. A snap-fit step is disposed on the surface of the fixed post.

The injection needle is inserted through the protective sleeve. A spring is clamped between the protective sleeve and the needle hub such that the protective sleeve is axially movable relative to the needle hub to expose or cover a needle tip of the injection needle. A protruding edge is disposed on the outer wall of the protective sleeve. A first limiting step and a second limiting step are disposed on the inner wall of the protective sleeve along a direction in which the injection needle penetrates. The inner diameter of the second limiting step is smaller than the inner diameter of the first limiting step.

The needle cap is fixedly assembled with the needle hub and is configured to enclose the protective sleeve within the needle cap. The needle cap is provided with a through-hole allowing the protective sleeve to extend out. The through-hole is configured to limit the protruding edge.

The limiting block is located in an inner cavity of the protective sleeve and is sleeved on the injection needle. A first end of the limiting block is provided with a claw that engages with the snap-fit step, and a second end of the limiting block cooperates with the first limiting step or the second limiting step to stop the protective sleeve.

Optionally, before use, the second end of the limiting block is restricted by the second limiting step, and the protective sleeve is capable of driving the limiting block to retract into the needle cap, thereby exposing the needle tip of the injection needle; during use, the limiting block engages with the fixed post via a snap-fit such that the limiting block is secured by the needle hub; and after use, the protective sleeve extends out of the needle cap to cover the needle tip, the second end of the limiting block disengages from the second limiting step and reaches the first limiting step to be restricted by the first limiting step, the first end of the limiting block abuts against the needle hub, and the protective sleeve is not capable of retracting into the needle cap again.

Optionally, the second end of the limiting block is provided with multiple expanded elastic pieces, and the multiple elastic pieces are only capable of undergoing inward elastic deformation under compression such that the limiting block can only move from the second limiting step back to the first limiting step and cannot move from the first limiting step forward to the second limiting step.

Optionally, the first end of the limiting block is provided with multiple notch grooves that allow elastic deformation of the end of the limiting block.

Optionally, the fixed post is configured as a tapered shape with a gradually increasing size from the head end of the fixed post to the snap-fit step.

Optionally, a guide slope is disposed between the second limiting step and the first limiting step.

Optionally, the protective sleeve is a split structure including a sleeve body and a retaining ring disposed at a port of the sleeve body, the sleeve body is in an interference fit with the retaining ring, the first limiting step is disposed on the retaining ring, and the second limiting step is disposed on the sleeve body.

Optionally, the inner wall of the needle cap is provided with a circumferential groove and multiple circumferentially evenly distributed guide grooves, and an outer wall of the needle hub is provided with a circumferential ridge cooperating with the circumferential groove and multiple axial ridges cooperating with the multiple guide grooves.

Optionally, a housing is also included. The housing is configured to enclose the needle cap and cover the protective sleeve and the needle hub within the housing. A tear-off seal configured as a cap is disposed on an opening of the housing.

Optionally, at least one of the inner wall of the housing or the outer wall of the needle cap is provided with a protruding ridge such that the housing is in an interference fit with the needle cap.

### BRIEF DESCRIPTION OF DRAWINGS

FIG. 1 is an external view of a safety insulin pen needle according to an embodiment of this application.
FIG. 2 is an exploded view of a safety insulin pen needle according to an embodiment of this application.
FIG. 3 is a view of a needle hub in a safety insulin pen needle according to an embodiment of this application.
FIG. 4 is a view of a needle cap in a safety insulin pen needle according to an embodiment of this application.
FIG. 5 is a view of a limiting block in a safety insulin pen needle according to an embodiment of this application.
FIG. 6 is a view of a safety insulin pen needle before use according to an embodiment of this application.
FIG. 7 is a view of a safety insulin pen needle during use according to an embodiment of this application.
FIG. 8 is a view of a safety insulin pen needle after use according to an embodiment of this application.

### Reference list

- 1: needle hub
- 11: injection needle
- 12: fixed post
- 13: snap-fit step
- 14: circumferential ridge
- 15: axial ridge
- 2: protective sleeve
- 21: protruding edge
- 22: first limiting step
- 23: second limiting step
- 24: sleeve body
- 25: retaining ring
- 26: guide slope
- 3: needle cap
- 31: through-hole
- 32: circumferential groove
- 33: guide groove
- 34: protruding ridge
- 4: limiting block
- 41: claw
- 42: notch groove
- 43: elastic piece
- 5: housing
- 51: tear-off seal
- 6: spring

### DETAILED DESCRIPTION

Embodiments of this application are described below. Examples of the embodiments are illustrated in the drawings, where the same or similar reference numerals indicate the same or similar elements or elements having the same or similar functions. The embodiments described below with reference to the drawings are exemplary, intended to explain this application, and not to be construed as limiting this application.

In the description of this application, unless otherwise expressly specified and limited, a term "connected to each other", "connected", or "secured" is to be construed in a broad sense, for example, as securely connected or detachably connected; mechanically connected or electrically connected; directly connected to each other or indirectly connected to each other via an intermediary; or internally connected between two components or interaction relations between two components. For those of ordinary skill in the art, specific meanings of the preceding terms in this application may be construed according to specific circumstances.

In the description of this application, unless otherwise expressly specified and limited, when a first feature is described as "above" or "below" a second feature, the first feature and the second feature may be in direct contact or be in contact via another feature between the two features instead of being in direct contact. Moreover, when the first feature is "on", "above", or "over" the second feature, the first feature is right on, above, or over the second feature, or the first feature is obliquely on, above, or over the second feature, or the first feature is simply at a higher level than the second feature. When the first feature is "under", "below", or "underneath" the second feature, the first feature is right under, below, or underneath the second feature, or the first feature is obliquely under, below, or underneath the second feature, or the first feature is simply at a lower level than the second feature.

The technical solutions of this application are described hereinafter in conjunction with drawings and embodiments.

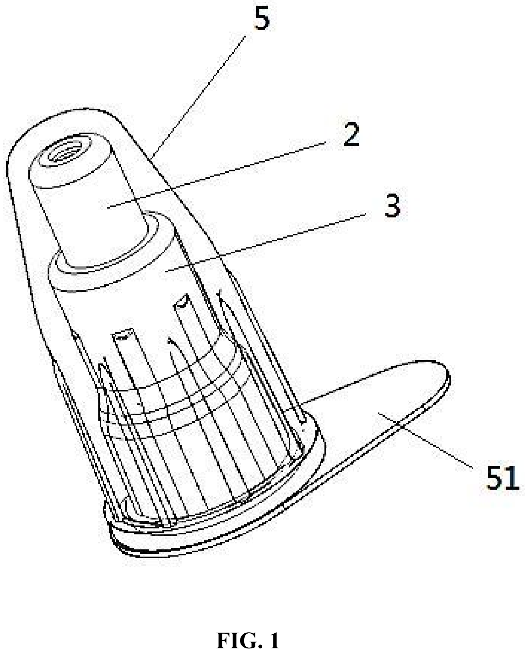

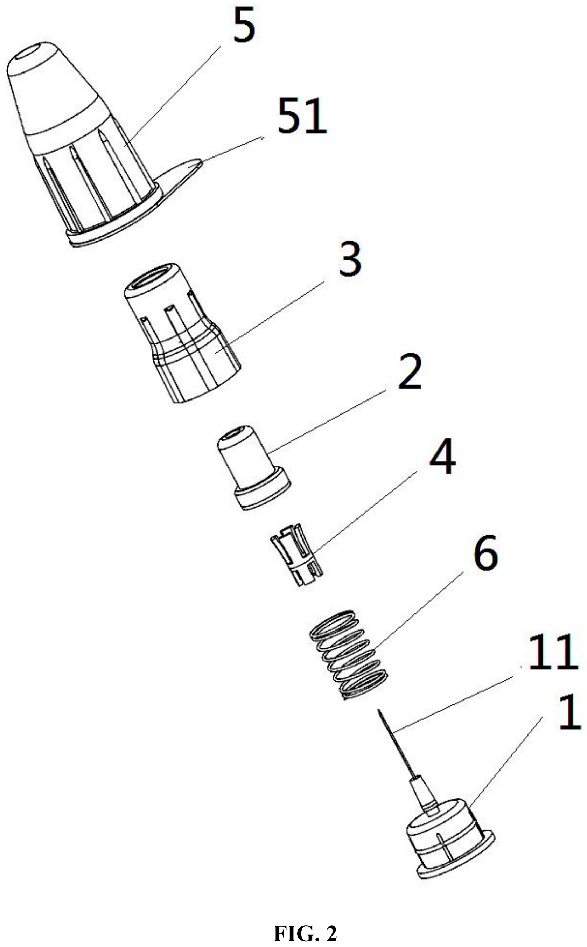

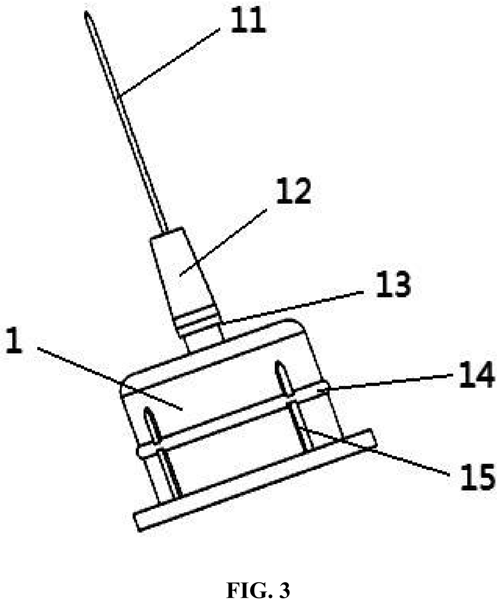

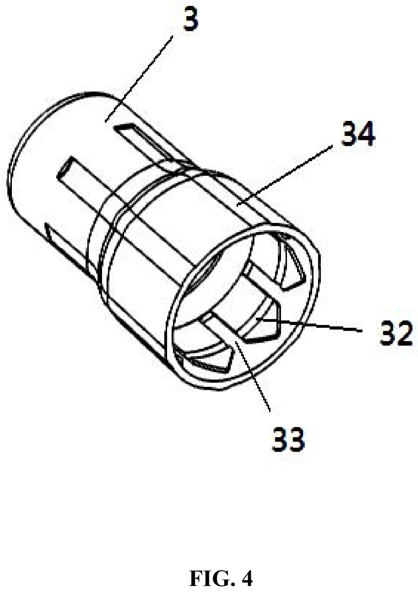

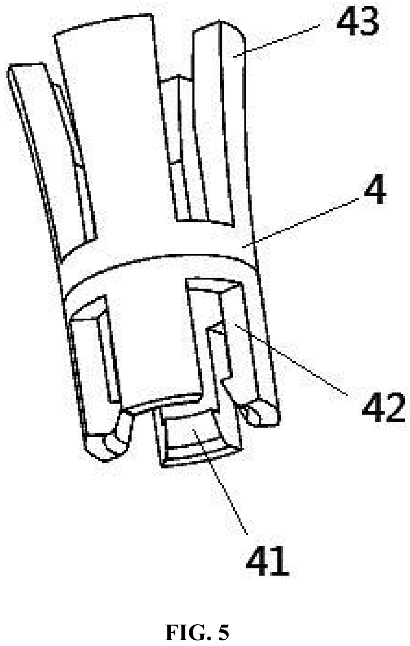

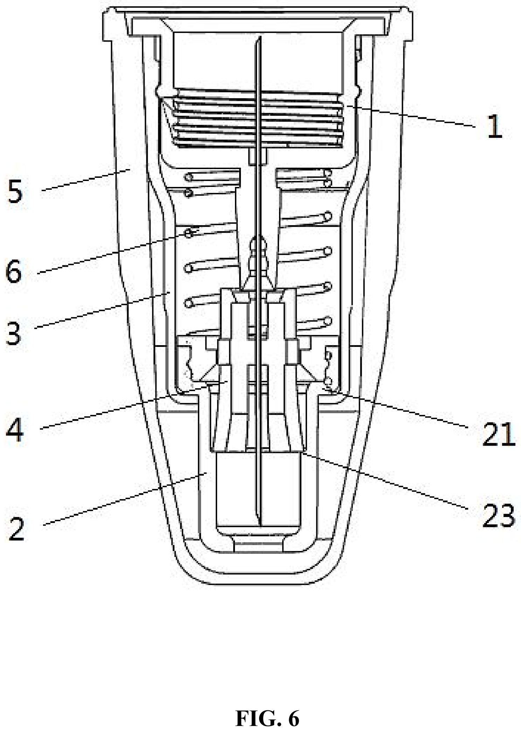

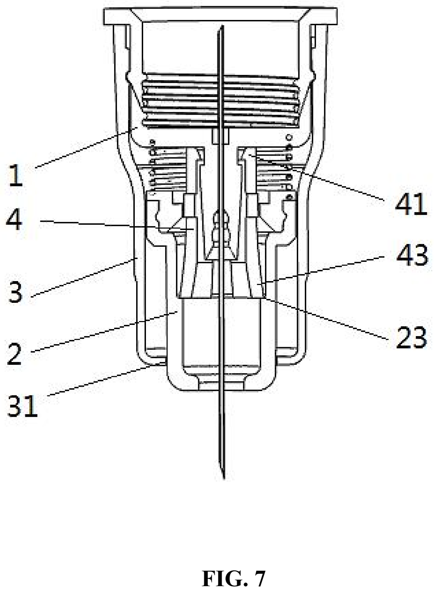

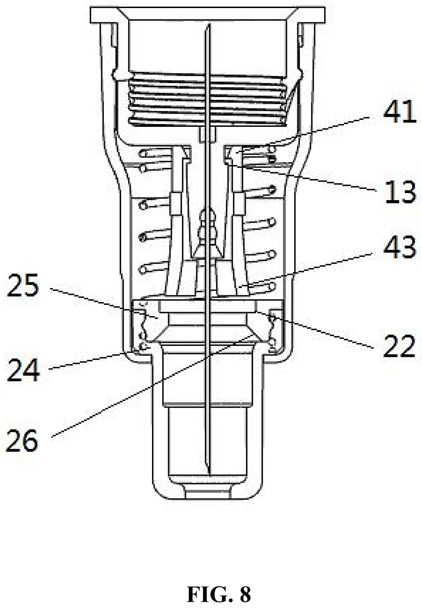

With reference to FIG. 1 to FIG. 8, this embodiment provides a safety insulin pen needle. The safety insulin pen needle includes a needle hub 1, a protective sleeve 2, a needle cap 3, a limiting block 4, and a housing 5.

A fixed post 12 protruding along with an injection needle 11 is disposed on the needle hub 1. A snap-fit step 13 is disposed on the surface of the fixed post 12. Optionally, the fixed post 12 is configured as a tapered shape with a gradually increasing size from the head end of the fixed post 12 to the snap-fit step 13, facilitating subsequent insertion.

The injection needle 11 is inserted through the protective sleeve 2. A spring 6 is interposed between the protective sleeve 2 and the needle hub 1 such that the protective sleeve 2 is axially movable relative to the needle hub 1 to expose or cover the needle tip of the injection needle 11. A protruding edge 21 is disposed on the outer wall of the protective sleeve 2. A first limiting step 22 and a second limiting step 23 are disposed on the inner wall of the protective sleeve 2 along a direction in which the injection needle 11 penetrates. The inner diameter of the second limiting step 23 is smaller than the inner diameter of the first limiting step 22.

The protective sleeve 2 may be integral or split. For ease of molding, this embodiment exemplifies a split component. The split component includes a sleeve body 24 and a retaining ring 25 disposed at the port of the sleeve body 24. The sleeve body 24 is in an interference fit with the retaining ring 25. The first limiting step 22 is disposed on the retaining ring 25, and the second limiting step 23 is disposed on the sleeve body 24.

Additionally, the first limiting step 22 may be formed directly on the second limiting step 23 or molded separately. When the first limiting step 22 is molded separately, a guide slope 26 may be disposed between the second limiting step 23 and the first limiting step 22 to facilitate movement of the limiting block 4 from the second limiting step 23 to the first limiting step 22.

The needle cap 3 is fixedly assembled with the needle hub 1 and encloses the protective sleeve 2 within the needle cap 3. The needle cap 3 is provided with a through-hole 31 allowing the protective sleeve 2 to extend out. The through-hole 31 limits the protruding edge 21 to restrict the telescopic movement of the protective sleeve 2 within the needle cap 3.

The inner wall of the needle cap 3 is provided with a circumferential groove 32 and multiple evenly distributed guide grooves 33 in the circumference, and the outer wall of the needle hub 3 is provided with a circumferential ridge 14 cooperating with the circumferential groove 32 and multiple axial ridges 15 cooperating with the multiple guide grooves 33 to ensure accurate and reliable assembly of the needle cap 3 with the needle hub 1.

The limiting block 4 is located in an inner cavity of the protective sleeve 2 and is sleeved on the injection needle 11. A first end of the limiting block 4 is provided with a claw 41 that engages with the snap-fit step 13, and a second end of the limiting block 4 cooperates with the first limiting step 22 or the second limiting step 23 to stop the protective sleeve 2.

The first end of the limiting block 4 is provided with multiple notch grooves 42 that allow elastic deformation of the end of the limiting block 4 to facilitate insertion of the fixed post 12. The second end of the limiting block 4 is provided with multiple expanded elastic pieces 43. The multiple elastic pieces 43 are only capable of undergoing inward elastic deformation under compression, such that the limiting block 4 can only move from the second limiting step 23 to the first limiting step 22 and cannot move from the first limiting step 22 to the second limiting step 23.

The housing 5 encloses the needle cap 3 and also covers the protective sleeve 2 and the needle hub 1 within the housing 5. A tear-off seal 51 configured as a cap is disposed on an opening of the housing 5.

At least one of the inner wall of the housing 5 or the outer wall of the needle cap 3 is provided with a protruding ridge 34 such that the housing 5 is in an interference fit with the needle cap 3, thereby ensuring structural stability of the product upon delivery.

The specific use process and principle of the safety insulin pen needle are as follows:

Before use, the tear-off seal 51 is removed, and the housing 5 is taken off. At this point, the second end of the limiting block 4 is restricted by the second limiting step 23, the needle tip of the injection needle 11 is covered by the protective sleeve 2, and the protective sleeve 2 can drive the limiting block 4 to perform telescopic movement.

During use, the injection needle 11 is inserted into an injection site, and the protective sleeve 2 is pressed into the needle cap 3, fully exposing the injection needle 11. During this process, the first end of the limiting block 4 engages with the fixed post 12 via a snap-fit such that the limiting block 4 is secured by the needle hub 1.

After use, the injection needle 11 is withdrawn, and the protective sleeve 2, driven by the spring 6, extends outward to cover the needle tip of the injection needle 11. Simultaneously, the second end of the limiting block 4 disengages from the second limiting step 23 and reaches the first limiting step 22, and is restricted by the first limiting step 22.

Consequently, if the protective sleeve 2 is pressed again, the second end of the limiting block 4 abuts against the protective sleeve 2, and the first end of the limiting block 4 abuts against the needle hub 1, thereby preventing the protective sleeve 2 from retracting into the needle cap 3 again.

In summary, the preceding safety insulin pen needle is designed with a protective sleeve, a needle hub, and a limiting block that cooperate with each other such that during the injection process, as the protective sleeve retracts and extends, a structural lock is achieved, preventing the protective sleeve from retracting again. In this manner, reuse of the injection needle is avoided without requiring additional operations, and safety and convenience of use are improved.

## Claims

1. A safety insulin pen needle, comprising:
a needle hub, wherein a fixed post protruding along with an injection needle is disposed on the needle hub, and a snap-fit step is disposed on a surface of the fixed post;
a protective sleeve, wherein the injection needle is inserted through the protective sleeve, a spring is clamped between the protective sleeve and the needle hub such that the protective sleeve is axially movable relative to the needle hub to expose or cover a needle tip of the injection needle, a protruding edge is disposed on an outer wall of the protective sleeve, a first limiting step and a second limiting step are disposed on an inner wall of the protective sleeve along a direction in which the injection needle penetrates, and an inner diameter of the second limiting step is smaller than an inner diameter of the first limiting step;
a needle cap, wherein the needle cap is fixedly assembled with the needle hub and is configured to enclose the protective sleeve within the needle cap, the needle cap is provided with a through-hole allowing the protective sleeve to extend out, and the through-hole is configured to limit the protruding edge; and
a limiting block, wherein the limiting block is located in an inner cavity of the protective sleeve and is sleeved on the injection needle, a first end of the limiting block is provided with a claw engaging with the snap-fit step, and a second end of the limiting block cooperates with the first limiting step or the second limiting step to stop the protective sleeve.

2. The safety insulin pen needle according to claim 1, wherein
before use, the second end of the limiting block is restricted by the second limiting step, and the protective sleeve is capable of driving the limiting block to retract into the needle cap to expose the needle tip of the injection needle;
during use, the limiting block engages with the fixed post via a snap-fit such that the limiting block is secured by the needle hub; and
after use, the protective sleeve extends out of the needle cap to cover the needle tip, the second end of the limiting block disengages from the second limiting step and reaches the first limiting step to be restricted by the first limiting step, the first end of the limiting block abuts against the needle hub, and the protective sleeve is incapable of retracting into the needle cap again.

3. The safety insulin pen needle according to claim 1, wherein the second end of the limiting block is provided with a plurality of expanded elastic pieces, and the plurality of elastic pieces are only capable of undergoing inward elastic deformation under compression such that the limiting block is only capable of retracting from the second limiting step back to the first limiting step and is not capable of retracting from the first limiting step forward to the second limiting step.

4. The safety insulin pen needle according to claim 1, wherein the first end of the limiting block is provided with a plurality of notch grooves that allow elastic deformation of an end of the limiting block.

5. The safety insulin pen needle according to claim 1, wherein the fixed post is configured as a tapered shape with a gradually increasing size from a head end of the fixed post to the snap-fit step.

6. The safety insulin pen needle according to claim 1, wherein a guide slope is disposed between the second limiting step and the first limiting step.

7. The safety insulin pen needle according to claim 1, wherein the protective sleeve is a split structure comprising a sleeve body and a retaining ring disposed at a port of the sleeve body, the sleeve body is in an interference fit with the retaining ring, the first limiting step is disposed on the retaining ring, and the second limiting step is disposed on the sleeve body.

8. The safety insulin pen needle according to claim 1, wherein a circumferential groove and a plurality of guide grooves evenly distributed in a circumference of the needle cap are provided on an inner wall of the needle cap, and a circumferential ridge cooperating with the circumferential groove and a plurality of axial ridges cooperating with the plurality of guide grooves are provided on an outer wall of the needle hub.

9. The safety insulin pen needle according to claim 1, further comprising a housing, wherein the housing is configured to enclose the needle cap and cover the protective sleeve and the needle hub within the housing, and a tear-off seal configured as a cap is disposed on an opening of the housing.

10. The safety insulin pen needle according to claim 9, wherein at least one of an inner wall of the housing or an outer wall of the needle cap is provided with a protruding ridge such that the housing is in an interference fit with the needle cap.
